# EUROPEAN PATENT APPLICATION

(11) **EP 3 255 573 A1**
(43) Date of publication of application: **13.12.2017**
(21) Application number: 16200822.1
(22) Date of filing: 25.11.2016
(51) Int. Cl.: G06F 19/00

(54) **CLINICAL DECISION SUPPORTING ENSEMBLE SYSTEM AND CLINICAL DECISON SUPPORTING METHOD USING THE SAME**

(30) Priority: 10.06.2016 KR 20160072645; 28.10.2016 KR 20160142185
(71) Applicant: Electronics and Telecommunications Research Institute, Yuseong-gu Daejeon 34129 (KR)
(72) Inventor: HAN, Youngwoong, Daejeon (KR); CHOI, Jae Hun, Daejeon (KR); KIM, Young Won, Daejeon (KR); KIM, Minho, Daejeon (KR); LIM, Myung-eun, Daejeon (KR); JUNG, Ho-Youl, Daejeon (KR); LEE, Dong-Hun, Daejeon (KR); KIM, Dae Hee, Daejeon (KR)
(74) Representative: Betten & Resch

(57) **Abstract**

Provided are a clinical decision supporting ensemble system and method. Clinical prediction results for a patient obtained through machine learning and received from a plurality of external medical institutions are integrated to perform an ensemble prediction, so that not only a current condition of the patient but also a future process of an illness of the patient is predicted to assist a medical person in making a quick and correct medical decision.

## Description

### CROSS-REFERENCE TO RELATED APPLICATIONS

This U.S. non-provisional patent application claims priority under 35 U.S.C. § 119 of Korean Patent Applications No. 10-2016-0072645, filed on June 10, 2016, and No. 10-2016-0142185, filed on October 28, 2016, the entire contents of which are hereby incorporated by reference.

### BACKGROUND

The present disclosure herein relates to a clinical decision supporting ensemble system and a clinical decision supporting method using the same, and more particularly, to a supporting system and method in which results of clinical prediction about a patient's disease received from at least one medical institution are integrated, and on the basis of the integrated results, a patient-customized analysis to which patient's characteristics are reflected is performed to provide a guideline on diagnosis and treatment of a patient to medical personnel, so that when the medical personnel determine a diagnosis or treatment method for a user, medical personnel's determination is cross checked with the guideline so as to avoid a misdiagnosis, and quick and correct decisions can be made with respect to medical activities for accurate prediction, diagnosis, treatment, prevention, and future management about a patient's disease.

According to a typical system for treating and detecting a condition of a patient having a disease or illness, condition diagnosis and treatment for a patient are performed depending on experience, knowledge, carrier, information, and the like of an individual medical person who diagnoses and treats the patient.

In this system, a difference in experience, knowledge, carrier, information between medical persons may cause a difference in the quality of diagnosis and treatment, and due to this difference, patients may prefer specific hospitals, and thus a hospital polarization phenomenon may become more serious.

With the development of a medical technology and an information processing technology, various medical devices are being developed, data used in the medical field such as medical records are being digitized, and the amount of systematized and accumulated information about medical researches are exponentially increasing. Furthermore, as personal health information collecting devices linkable to smart devices are widely used, pieces of clinical information including personal health information increase enough to form big data.

Various and new diseases or illnesses have occurred due to global warming caused by the development of an industrial technology, an increase of threat to human bodies, or changes of life styles or habits.

Due to such a change of a medical environment, clinical information required to be referred to by medical personnel to diagnose and treat patients becomes various and complex. Therefore, when a patient is diagnosed and treated depending on an individual medical person, a medical accident may occur due to a misdiagnosis, causing serious physical or mental damage to the patient.

That is, in a medical environment of the present time, it is difficult to make an accurate diagnosis or establish a treatment strategy for a patient depending on experience or information of an individual medical person, and diagnosing or treating a new disease is limited.

To overcome the above-mentioned limitation, a clinical decision supporting system (CDSS) has been developed to assist a medical person in making a decision when diagnosing or treating a disease of a patient on the basis of clinical information of the patient, and the system is used in various medical fields.

In general, the clinical decision supporting system programs a medical diagnosis and treatment guideline according to a rule (more specifically, IF-THEN rule) to provide, to a doctor, a result of a guideline for a condition of a patient, and representatively, the clinical decision supporting system provides a drug utilization review (DUR) service for setting restrictions on a plurality of complex drugs when the drugs are prescribed so that drug interactions may be prevented.

The clinical decision supporting system assists a medical person in making a decision about a diagnosis, selection of a treatment method, or selection of a treatment drug for a patient on the basis of clinical information of the patient, so that the possibility of a misdiagnosis by the medical person may be minimized, and a diagnosis and a treatment may be performed more objectively. Accordingly, the difference in the quality of diagnosis and treatment between medical persons may be reduced so that an objective medical service may be provided to the patient.

The incidence of chronic diseases such as cardiovascular disorders or diabetes has recently been increased. Since the chronic diseases have a high probability of a sudden death due to acute exacerbation, prediction of an emergency situation, an early diagnosis of complications, and future care of a patient are very important.

However, a typical clinical decision supporting system does not provide a guideline on an early diagnosis or prediction of a progress of a disease, but only provides a function of administration warning or drug prescription, and obtains a result only within a determined rule, and is thus limited in making an accurate diagnosis or establishing a treatment strategy according to a progress of a disease by performing an overall management such as prediction, diagnosis, treatment, and prevention of a disease of a patient.

Furthermore, since the typical clinical decision supporting system is based on a result of repeatedly learning cases of the same patient, the typical clinical decision supporting system is unable to provide a reliable diagnosis or treatment method for a new patient having the same disease.

Moreover, clinical information required for diagnosing or treating patients is generated, accumulated, and managed in medical service providing institutions, such as hospitals or oriental medical clinics, in their own data formats, and on the basis of the clinical information, the medical service providing institutions establish and operate their own clinical decision supporting systems. Due to a lack of interoperation between the systems of the medical service providing institutions, pieces of newly discovered information about a specific disease cannot be used efficiently.

Therefore, the present disclosure provides a supporting system and method in which results of clinical prediction about similar cases of a specific patient are integrated on the basis of clinical information generated, accumulated, and managed in each medical institution through interoperation with a plurality of medical institutions by using an ensemble prediction system, and a patient-customized analysis to which patient's characteristics and the integrated clinical prediction results are applied is performed to provide a guideline on diagnosis and treatment of a patient to a medical person, so that quick and correct decisions can be made with respect to medical activities such as diagnosis, treatment, prescription, prevention, and future management about a patient's disease.

Furthermore, the present disclosure provides a system and method for preventing occurrence of an emergency situation by early predicting a dangerous situation related to a disease of a high-risk patient, such as cardiovascular disorders, through continuous monitoring and management of the disease of the patient by collecting biometric data of the patient using an IoT device.

Next, the prior art of the technical field to which the present disclosure belongs will be described, and then technical matters to be addressed by the present disclosure will be described.

Korean Patent No. 1558142 (October 8, 2015), which relates to a decision supporting system for medical personnel, discloses a system in which various data such as a result of examining a patient, a vital sign, patient's statements, and observations on a patient are applied to a decision making rule related to a treatment of a patient so that an appropriate treatment plan for a patient may be established.

Korean Patent Application Laid-open Publication No. 2009-0000196 (January 7, 2009), which relates to a clinical decision support device using homecare data and medical diagnosis/treatment information, discloses an automated clinical decision support device which enables quick diagnosis and primary care by using measurement data transmitted via a home-health network and various sensors and patient's medical data being managed in an existing medical institution.

According to the above-mentioned prior arts, only a condition of a patient at a specific time is detected using patient information collected in each hospital, and on the basis of the detected condition, diagnosis and treatment plans for the patient are established, but information for making a reliable clinical decision about diagnosis and treatment plans for a new future patient cannot be provided.

On the contrary, according to the present disclosure, a specific medical institution, which makes a clinical decision to diagnose and treat a patient, requests a result of clinical prediction about a condition of the patient from at least one external medical institution via a common ensemble prediction system, and each external medical institution provides, to the specific medical institution, a clinical prediction result obtained by individually predicting the condition of the patient on the basis of clinical information generated, accumulated, and managed by each external medical institution, and the specific medical institution provides a guideline on prediction, diagnosis, treatment, prevention, and future management about the condition of the patient on the basis of the clinical prediction result received from the external medical institutions and clinical information of the patient.

### SUMMARY

The present disclosure provides a system and method in which clinical prediction results for a patient respectively obtained in a plurality of medical institutions are integrated through interoperation between the medical institutions, and a diagnosis on the patient, to which characteristics of the patient are reflected, is performed and a future progress of a condition of the patient is predicted on the basis of the integrated clinical prediction results, so as to provide a guideline required for a medical person to make a decision about medical activities, thereby preventing a misdiagnosis by the medical person and assisting the medical person in making a correct and quick decision about a prediction, diagnosis, prevention, emergency situation, or efficient illness management regarding the condition of the patient.

The present disclosure also provides a system and method in which biometric data of a patient, such as a lifelog, is collected through an IoT device to continuously monitor and manage the condition of the patient, so that occurrence of an emergency situation or exacerbation of a disease of a high-risk patient may be predicted and prevented.

An embodiment of the inventive concept provides a clinical decision supporting ensemble system configured to provide clinical decision information by performing an ensemble prediction by integrating clinical prediction results provided from a plurality of medical institutions, medical information providing institutions, or combinations thereof.

In an embodiment, the ensemble prediction may be performed on the basis of the clinical prediction results obtained through machine learning using medical information learning big data of each of the plurality of medical institutions.

In an embodiment, the ensemble prediction may support a clinical decision by integrating a clinical prediction result based on machine learning of own medical information learning big data of a specific medical institution and clinical prediction results based on machine learning of medical information learning big data of each of one or more external medical institutions.

In an embodiment, the clinical decision information may be provided by: a knowledge base; a decision tree, a neural network, naive Bayes, or a combination thereof according to each person, illness, or a combination thereof; or a combination thereof.

In an embodiment, the machine learning may include an artificial intelligence technique for performing learning, inference, prediction, or a combination thereof in order to multi-dimensionally analyze big data including a medical record, a lifelog, or a combination thereof for a patient-centered medical service.

In an embodiment, the machine learning may be performed to output, as a clinical prediction result, highly reliable numerical information extracted or integrated through speeding up by a parallel cluster and quantization of medical information learning big data.

In an embodiment of the inventive concept, a clinical decision supporting ensemble system includes: a clinical decision supporting system configured to provide clinical decision information in response to a clinical decision request of a user; and an ensemble prediction system configured to provide a clinical prediction result in response to a request of the clinical decision supporting system, wherein the ensemble prediction system performs an ensemble prediction for a decision by integrating clinical prediction results provided from a plurality of medical institutions, medical information providing institutions, or combinations thereof.

In an embodiment, the clinical decision supporting ensemble system may further include a machine learning engine including a deep learning algorithm for performing learning by using big data from a clinical information database.

In an embodiment, the clinical information database may include hospital clinical information of an individual hospital and lifelog information of an outpatient.

In an embodiment, the machine learning engine may abstract features of medical information from big data including an electronic medical record (EMR), a personal health record (PHR), a medical image, lifelog information, or a combination thereof, and may extract a prediction model by learning the big data to early predict a dangerous situation of a disease, thereby improving reliability of a clinical prediction result.

In an embodiment, the ensemble prediction system may include: an interface configured to send a prediction request to ensemble prediction systems present in a plurality of external medical institutions and receive prediction results from the external medical institutions; and a cooperative hospital management unit configured to manage hospital information for cooperating with the external medical institutions.

In an embodiment, the institutions may be provided with the ensemble prediction system, a machine learning engine, and medical information learning big data.

In an embodiment, the clinical decision supporting ensemble system may collect biometric data of a patient including a lifelog of the patient to continuously monitor and manage a condition of the patient, and may provide the condition of the patient to an IoT device.

In an embodiment of the inventive concept, a clinical decision supporting method includes: performing an ensemble prediction by integrating a plurality of clinical prediction results; and providing clinical decision information with improved accuracy through the ensemble prediction.

In an embodiment, the ensemble prediction may be performed on the basis of the clinical prediction results obtained through machine learning using medical information learning big data of each of a plurality of medical institutions.

In an embodiment, the ensemble prediction may support a clinical decision by integrating a clinical prediction result based on machine learning of own medical information learning big data of a specific medical institution and clinical prediction results based on machine learning of medical information learning big data of each of one or more external medical institutions.

In an embodiment, the clinical decision information may be provided by: a knowledge base; a decision tree, a neural network, naive Bayes, or a combination thereof according to each person, illness, or a combination thereof; or a combination thereof.

In an embodiment, the machine learning may include an artificial intelligence technique for performing learning, inference, prediction, or a combination thereof in order to multi-dimensionally analyze big data including a medical record, a lifelog, or a combination thereof for a patient-centered medical service.

In an embodiment, the machine learning may be performed to output, as a clinical prediction result, highly reliable numerical information extracted or integrated through speeding up by a parallel cluster and quantization of medical information learning big data.

In an embodiment of the inventive concept, a clinical decision supporting method includes: providing clinical decision information in response to a clinical decision request of a user via a clinical decision supporting system; and providing a clinical prediction result in response to a request of the clinical decision supporting system via an ensemble prediction system, wherein the ensemble prediction system performs an ensemble prediction for a decision by integrating clinical prediction results provided from a plurality of medical institutions, medical information providing institutions, or combinations thereof.

In an embodiment, the clinical decision supporting method may further include performing learning by using big data from a clinical information database through a machine learning engine.

In an embodiment, the clinical information database may include hospital clinical information of an individual hospital and lifelog information of an outpatient.

In an embodiment, the machine learning engine may abstract features of medical information from big data including an electronic medical record (EMR), a personal health record (PHR), a medical image, lifelog information, or a combination thereof, and may extract a clinical prediction model by learning the big data to early predict a dangerous situation of a disease, thereby improving reliability of a clinical prediction result.

In an embodiment, the ensemble prediction system may include: an interface configured to send a prediction request to ensemble prediction systems present in a plurality of external medical institutions and receive prediction results from the external medical institutions; and a cooperative hospital management unit configured to manage hospital information for cooperating with the external medical institutions.

In an embodiment, the institutions may be provided with the ensemble prediction system, a machine learning engine, and medical information learning big data.

In an embodiment, the clinical decision supporting method may include collecting biometric data of a patient including lifelog information of the patient to continuously monitor and manage a condition of the patient, and providing the condition of the patient to an IoT device.

In an embodiment of the inventive concept, a clinical decision supporting method includes receiving prediction results generated by other medical institutions or medical information providing institutions by using, as an input, learning data provided from a specific medical institution or medical information providing institution, integrating the received prediction results and a prediction result generated autonomously by the specific medical institution or medical information providing institution to extract ensemble learning data, and performing ensemble learning by using the extracted ensemble learning data.

In an embodiment, the receiving the prediction results may include: generating, by the specific medical institution or medical information providing institution, learning data to provide the learning data to the other medical institutions or medical information providing institutions; and providing, to the specific medical institution or medical information providing institution, the prediction results obtained using the learning data as the input to a prediction model of the other medical institutions or medical information providing institutions.

In an embodiment, the ensemble learning may include: performing pre-training by using the prediction results of each of the other medical institutions or medical information providing institutions; and performing fine tuning by using illness information in addition to the prediction results.

In an embodiment of the inventive concept, a clinical decision supporting method includes integrating an own prediction result generated by a specific medical institution or medical information providing institution by using clinical data of a specific patient and prediction results generated by other medical institutions or medical information providing institutions by using the clinical data of the specific patient as an input, and performing an ensemble prediction using the integrated prediction results.

In an embodiment of the inventive concept, a clinical decision supporting ensemble system is configured to receive prediction results generated by other medical institutions or medical information providing institutions by using, as an input, learning data provided from a specific medical institution or medical information providing institution, integrate the received prediction results and a prediction result generated autonomously by the specific medical institution or medical information providing institution to extract ensemble learning data, and perform ensemble learning by using the extracted ensemble learning data.

### BRIEF DESCRIPTION OF THE FIGURES

The accompanying drawings are included to provide a further understanding of the inventive concept, and are incorporated in and constitute a part of this specification. The drawings illustrate exemplary embodiments of the inventive concept and, together with the description, serve to explain principles of the inventive concept. In the drawings:
FIG. 1 is a conceptual diagram illustrating a clinical decision supporting ensemble system according to an embodiment of the inventive concept;
FIG. 2 is a block diagram illustrating a process of providing clinical decision information according to an embodiment of the inventive concept;
FIG. 3 is a block diagram illustrating a configuration of a clinical decision supporting ensemble system according to an embodiment of the inventive concept;
FIG. 4 is a diagram illustrating a process of performing machine learning through a machine learning engine in each medical institution according to an embodiment of the inventive concept;
FIG. 5 is a diagram illustrating a process of performing ensemble learning for an ensemble prediction in an ensemble prediction system according to another embodiment of the inventive concept;
FIG. 6 is a diagram illustrating a process of outputting an ensemble prediction result for a specific patient through ensemble learning to provide clinical decision information to a user according to another embodiment of the inventive concept;
FIG. 7 is a block diagram illustrating a configuration of a clinical decision supporting ensemble system according to another embodiment of the inventive concept; and
FIG. 8 is a flowchart illustrating a procedure of generating clinical decision information to provide the clinical decision information to a terminal of a medical person according to an embodiment of the inventive concept.

### DETAILED DESCRIPTION

Hereinafter, embodiments of the inventive concept will be described in detail with reference to the accompanying drawings. Like reference numerals refer to like elements throughout.

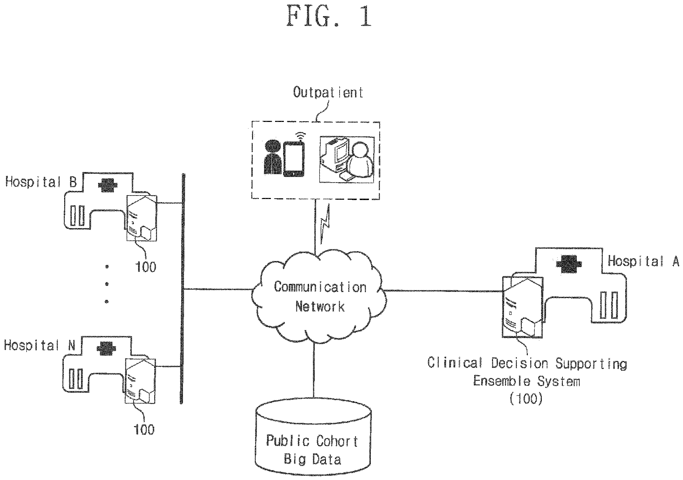

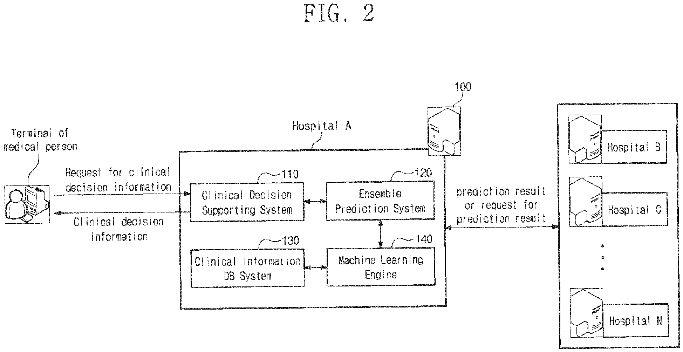

FIG. 1 is a conceptual diagram illustrating a clinical decision supporting ensemble system according to an embodiment of the inventive concept, and FIG. 2 is a block diagram illustrating a process of providing clinical decision information according to an embodiment of the inventive concept.

As illustrated in FIGS. 1 and 2, clinical decision supporting ensemble systems 100 are established in a plurality of medical institutions, medical information providing institutions, or combinations thereof, and the institutions may interwork with each other through the clinical decision supporting ensemble systems 100.

The clinical decision supporting ensemble system 100 includes a clinical decision supporting system 110 which provides clinical decision information to a user (i.e., a medical person) who treats a patient, a machine learning engine 140 which performs machine learning on the basis of big data for medical information learning stored in a clinical information database 130 to generate a clinical prediction model for each illness, and an ensemble prediction system 120 which outputs a clinical prediction result of the patient on the basis of the generated clinical prediction model.

A medical person who treats a specific patient requests clinical decision information from the clinical decision supporting system 110 in order to correctly determine and perform medical activities such as a diagnosis of an illness of a patient, selection of a treatment method, and selection of a treatment drug.

When the clinical decision supporting system 110 receives the request for clinical decision information from the medical person, the clinical decision supporting system 110 requests an ensemble prediction result of the patient from the ensemble prediction system 120, and the ensemble prediction system 120 requests the clinical prediction result from a plurality of external medical institutions via a communication network. Here, the ensemble prediction system 120 provides the clinical information of the patient to the external medical institutions, and each external medical institution performs clinical prediction of the patient on the basis of the clinical information of the patient.

Hereinafter, the term "medical institution" is used to represent either of a medical institution such as a hospital or an oriental medical clinic and a medical information providing institution such as National Health Insurance Service or Health Insurance Review & Assessment Service.

The clinical information of the patient provided to the external medical institutions lacks personal information of the patient, such as the name, address, or resident registration number of the patient, but includes an electronic medical record (EMR) including the age of the patient, the type of an illness, patient's tastes (e.g., smoking, drinking, etc.), prescription, or a combination thereof, a medical image, a lifelog, or a combination thereof. The clinical information may be changed according to an illness of the patient.

The clinical prediction represents prediction of a current state of the illness of the patient and a future progress of the illness.

When the clinical decision supporting ensemble system 100 receives, from another medical institution, a request for a clinical prediction result, the clinical decision supporting ensemble system 100 performs the clinical prediction of the patient by inputting the clinical information of the patient to the clinical prediction model generated by the machine learning engine 140, and outputs a result of the clinical prediction.

The clinical prediction is performed in each medical institution which has received the request for a clinical prediction result, and each medical institution performs the clinical prediction on the basis of clinical information generated, accumulated, stored, and managed by each medical institution, and transmits a result of the clinical prediction to a medical institution which has made the request. The clinical information generated, accumulated, stored, and managed by each medical institution is time-series big data which is medical information for each patient including hospital clinical information and PHR collected from a plurality of patients.

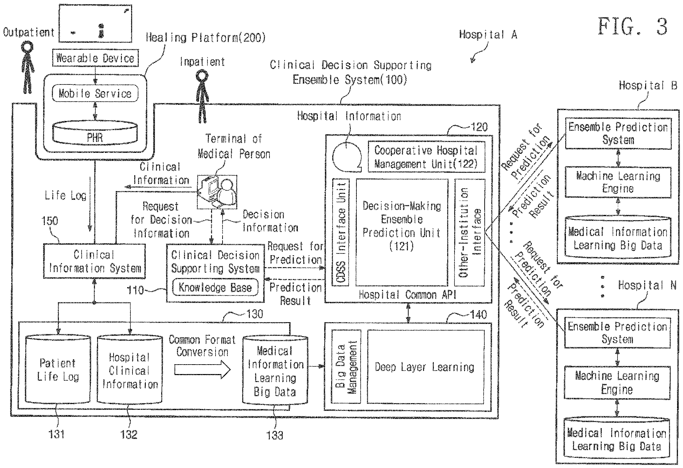

The PHR and the hospital clinical information are described in detail below with reference to FIG. 3.

The machine learning engine 140 extracts the clinical prediction model by performing machine learning on the basis of its own clinical information in each medical institution. Furthermore, the machine learning engine 140 may perform machine learning using not only its own clinical information but also public cohort big data for public clinical information provided from a medical information providing institution such as National Health Insurance Service or Health Insurance Review & Assessment Service, and this operation may ensure extraction of an accurate and reliable clinical prediction model.

The machine learning engine 140 may be implemented by applying different machine learning techniques according to characteristics of medical institutions, such as a specialized field or an application scope.

When a single machine learning technique is applied to the machine learning engine 140, an optimal result may not be derived for all cases. Therefore, a clinical prediction model capable of providing improved accuracy is extracted using a combination of a plurality of machine learning techniques, so that a more accurate and reliable clinical prediction result may be output.

The clinical predication model extracted by the machine learning engine 140 receives, as an input, clinical information of a specific patient from the ensemble prediction system 120 of the medical institution which has requested a clinical prediction result, and outputs a clinical prediction result obtained by predicting a future progress of an illness of the patient. That is, the clinical prediction result is information output on the basis of similar cases of the clinical information of the patient, and is numerical information obtained by predicting the progress of the illness of the patient.

Furthermore, the ensemble prediction system 120 transmits the output clinical prediction result to the ensemble prediction system of the medical institution which has requested the clinical prediction result. Here, an opinion of a medical specialist about a corresponding illness or a guideline such as a treatment method or a prescription proposed by a corresponding medical institution according to the clinical prediction result may be transmitted together with the clinical prediction result.

The ensemble prediction system 120 which has received the clinical prediction results from the external medical institutions performs a decision-making ensemble prediction by integrating the received clinical prediction results, and provides a result of the ensemble prediction to the clinical decision supporting system 110.

The ensemble prediction represents predicting a patient's current condition and a future progress of an illness to which characteristics of the patient are applied in order to assist with a clinical decision of a medical person, by integrating, on the basis of the clinical information of the patient, at least one clinical prediction result received from at least one external medical institution with a clinical prediction result output from a specific medical institution which has requested the at least one clinical prediction result. This process enables a more accurate and reliable prediction of a current condition of a patient and a future progress of an illness through interworking with a plurality of external medical institutions, particularly, medical institutions specializing in the illness of the patient, in comparison with a process in which a single medical institution performs a clinical prediction for a specific patient. Furthermore, pieces of new and valuable information about the illness discovered by the external medical institutions may be used, so that a highly reliable ensemble prediction result may be obtained.

The degree of accuracy of the machine learning engine 140 included in the clinical decision supporting ensemble system 100 of each medical institution is improved as the amount of big data machine-learned by the machine learning engine 140 increases.

Furthermore, the clinical decision supporting system 110 provides clinical decision information to a medical person using a prediction result received from the ensemble prediction system 120.

The clinical decision information includes a diagnosis on a current condition of a patient and a future progress of the condition of the patient, a configuration for an optimal treatment method such as a drug prescription or surgery, a prescription, a warning on the prescription, or a combination thereof. This information may assist a medical person in making a decision about a suitable and accurate medical activity by providing a guideline on the current condition of the patient and the future progress of the condition when the medical person performs medical activities such as diagnosing and treating an illness on the basis of clinical information collected from the patient, so that the possibility of a misdiagnosis by the medical person may be minimized, and the patient may receive objective medical activities.

The clinical decision supporting ensemble system 100 receives biometric information of an outpatient from an IoT (internet of things) device of the outpatient to monitor a condition of the outpatient, and performs detection and predictive diagnosis of a condition change of the outpatient on the basis of a result of ensemble prediction of the condition of the outpatient according to the biometric information so that an emergency situation may be detected early and addressed quickly. Furthermore, the clinical decision supporting ensemble system 100 provides the clinical prediction result to the IoT device.

The IoT device includes a biosensor and a wireless communication terminal. The biosensor, which is a wearable device attached to a body of the outpatient, collects lifelog information including various pieces of biometric information of the outpatient, such as a blood pressure, blood glucose, hormones, electrocardiogram, exercise amount information, cholesterol level, etc.

Furthermore, the biosensor operates in association with a mobile service to transmit, in real time or periodically, the various pieces of biometric information of the outpatient to the clinical decision supporting ensemble system 100. In this manner, the clinical decision supporting ensemble system 100 may monitor and manage the condition of the patient in real time or periodically, and may predict and prevent occurrence of an emergency situation or exacerbation of a disease of a high-risk patient, such as cardiovascular disorders, by performing an ensemble prediction on the basis of the lifelog information including the biometric information, and may provide a result of the ensemble prediction to the wireless communication terminal.

FIG. 3 is a block diagram illustrating a configuration of the clinical decision supporting ensemble system 100 according to an embodiment of the inventive concept.

As illustrated in FIG. 3, the clinical decision supporting ensemble system 100 includes a clinical information system 150 which collects lifelog information of an outpatient and clinical information of an inpatient, the clinical information DB system 130 which stores and manages the collected lifelog information and clinical information, the machine learning engine 140 which performs deep layer learning of the stored lifelog information and clinical information, the ensemble prediction system 120 which performs a decision-making ensemble prediction, and the clinical decision supporting system 110 which generates clinical decision information about medical activities for a patient on the basis of the ensemble prediction, and provides the clinical decision information to a medical person.

The outpatient is provided with a wearable-type biosensor to measure, collect, and store lifelog information including biometric information and activity information of the outpatient in real time or periodically, and transmits the collected lifelog information to the clinical information system 150 in real time or periodically, wherein this transmission is performed through a mobile service associated with the biosensor.

The mobile service provides a healing platform 200 to the outpatient, and the outpatient may transmit the lifelog information to the clinical information system 150 through the healing platform 200.

The healing platform 200 stores a personal health record (PHR) of the outpatient in a personal storage so that the PHR may be managed by the outpatient for himself or herself. The personal storage may be a local repository or a personal cloud repository.

The personal health record includes the lifelog information and an electronic medical record computerized and stored in a medical institution, and the outpatient may receive, through the healing platform 200, the electronic medical record of the outpatient from a medical institution from which the outpatient has received a medical treatment. In this manner, the outpatient may manage the personal health record for himself or herself, and may receive medical services provided from a plurality of medical institutions, consistently and continuously without overlaps.

The lifelog information is collected from not only outpatients but also inpatients.

In the case where the clinical information and the lifelog information have been collected from the same patient, the clinical information system 150 may store the collected clinical information and lifelog information in association with the patient.

The clinical information system 150 includes an order communication system (OCS) (not shown), a picture archive and communication system (PACS) (not shown), an electronic medical record (EMR) system (not shown), and a laboratory information system (LIS) (not shown).

The OCS delivers a prescription issued by a medical person after diagnosing a patient to a relevant medical department via a short-range communication network so as to organically connect the prescription by the medical person for the patient or an activity of nursing the patient by a nurse to each department, so that an error that may occur during a prescription delivery process may be reduced by reducing miscommunication between doctors, nurses, and other relevant departments. Accordingly, the clinical information system 150 stores information (OCS information) about the prescription by the medical person in a hospital clinical information database 132 in association with each patient.

The PACS is a medical image integrated management system which obtains medical image information (PACS information) of a patient from a medical imaging device, and then stores, manages, and transmits the information via a high-speed network, so that a typical film-based hospital task environment may be digitized so as to efficiently manage medical image information. Accordingly, the clinical information system 150 stores the medical image information in the hospital clinical information database 132 in association with each patient.

The EMR system represents a system which records all matters related to an illness of a patient in a digitized electronic medical record in which all matters related to the illness of the patient and information about an examination or a treatment provided to the patient by a medical institution and a result of the examination or treatment are correctly recorded. The electronic medical record is used as base data for providing a consistent and continuous medical service to the patient. That is, since the electronic medical record is digitized to be immediately used by a medical person through a terminal when a patient comes a hospital to receive a medical diagnosis or treatment, problems due to a waiting time may be resolved, convenience may be given to the patient, and the reliability of a medical institution may be increased, so that a patient-centered medical service may be provided. Accordingly, the clinical information system 150 stores the electronic medical record in the hospital clinical information database 132 in association with each patient.

The LIS represents a system in which pieces of examination equipment of a medical institution are automated so that information about results of examinations performed by the examination equipment is provided to patients and medical persons correctly and quickly. The examination result information may be recorded in the electronic medical record or may be stored independently. Accordingly, the clinical information system 150 stores the examination result information in the hospital clinical information database 132 in association with each patient.

The clinical information DB system 130 includes a clinical information database (not shown), which includes a patient lifelog database 131 for storing and managing lifelog information collected from outpatients or inpatients, the hospital clinical information database 132 for storing and managing hospital clinical information collected from the clinical information system 150, and a medical information learning big data database 133 for storing and managing medical information learning big data obtained by converting the lifelog information and the hospital clinical information into a common format so that the lifelog information and the hospital clinical information may be easily learned by the machine learning engine 140.

The medical information learning big data, which is medical information including the hospital clinical information including the EMR system, the PACS, the OCS, and the LIS, or a combination thereof, represents big data to be learned by the machine learning engine 140. The machine learning engine 140 performs learning by automatically extracting and abstracting features of the above-mentioned medical information, such as numerical values (age, biometric data, diabetes level, or electrocardiogram value), a treatment process, a prescription, tastes (smoking or drinking), or a change in a condition of a patient.

The lifelog database 131 stores outpatients' lifelog information collected in real time or periodically by the clinical information system 150.

The hospital clinical information database 132 may receive and store hospital clinical information including OCS information (prescription information) for each patient, PACS information (medical image information) for each patient, and LIS information (examination result information) for each patient from the clinical information system 150, wherein the LIS information may be recorded in the electronic medical record.

The lifelog information and the hospital clinical information are stored in association with each patient.

The clinical information DB system 130 generates the medical information learning big data by converting the lifelog information and the hospital clinical information into a common format and stores the medical information learning big data in the medical information learning big data database 133, so that the machine learning engine 140 may integrally learn the lifelog information and the hospital clinical information.

The common format, which is an HL7 standard, has a type of virtual medical record (VMR), extendible VMR (eVMR), or Arden syntax so as to be commonly used by medical institutions. The common format may include not only the VMR, eVMR, or Arden syntax but also any other standard data formats that may be commonly used by a plurality of medical institutions.

The medical information learning big data may be converted and managed in a medical institution's own format, and clinical prediction results received from a plurality of external medical institutions may be converted into the medical institution's format so as to be used.

The machine learning engine 140 machine-learns the stored medical information learning big data through deep learning, and extracts a clinical prediction model which is a highly reliable multilayer cognitive network on the basis of the medical information learning big data. Each layer included in the network repeatedly performs machine learning by automatically extracting and abstracting medical features (e.g., age, biometric data, diabetes level, electrocardiogram value, a treatment process, a prescription, tastes (smoking or drinking), a change in a condition of a patient, etc.) of an illness on the basis of the medical information learning big data. In this manner, the clinical prediction model performs a clinical prediction about a future progress of the illness to output a clinical prediction result. That is, the machine learning engine 140 abstracts features of clinical information which is medical information including an electronic medical record, a PHR including lifelog information, a medical image, or a combination thereof so as to elaborate the medical information learning big data, and extracts a clinical prediction model so as to early predict a dangerous situation and a progress of an illness or disease of a specific patient, thereby improving the reliability of the clinical prediction result.

The machine learning includes an artificial intelligence technique for performing learning, inference, prediction, or a combination thereof in order to multi-dimensionally analyze big data including a medical record, lifelog information, or a combination thereof for a patient-centered medical service, so that a reliable clinical prediction result may be output.

As the amount of the medical information learning big data increases, the machine learning engine 140 may generate a more correct and reliable clinical prediction result, and whenever data is added to the medical information learning big data, the machine learning engine 140 repeatedly performs machine learning so as to automatically update the clinical prediction model so that a more correct clinical prediction result may be output.

The machine learning performed in the machine learning engine 140 enables high-speed output of a clinical prediction result using a parallel cluster, and outputs, as a clinical prediction result, highly reliable numerical information extracted or integrated through quantization of the medical information learning big data.

Furthermore, the medical information learning big data is medical information big data having time-series characteristics, from which personal information of a patient, such as a name, a resident registration number, an address, or the like, has been deleted.

The machine learning engine 140 may perform machine learning for each illness (e.g., diabetes or cardiovascular disorders) or may perform simultaneous machine learning by combining a plurality of illnesses (e.g., diabetes and diabetic complications or cardiovascular disorders and other chronic illnesses). This is because a patient may have not only a single illness but also other illnesses simultaneously. For example, it is a common case that a patient having diabetes also have diabetic complications.

Accordingly, at least one clinical prediction model is generated by the machine learning engine 140, and may be variously generated according to a specialized field and characteristics of each medical institution. For example, in the case where a specific medical institution specializes in cancer, a clinical prediction model may be generated according to the type of a cancer.

Furthermore, an input of a generated clinical prediction model is clinical information from which personal information of a specific patient has been deleted, and an output of the clinical prediction model is a clinical prediction result obtained by predicting a future progress of an illness of the patient.

The ensemble prediction system 120 includes a decision-making ensemble prediction unit 121 which performs an ensemble prediction for making a decision, a cooperative hospital management unit 122 which manages hospital information for cooperation with a plurality of external medical institutions, an other-institution interface unit for requesting and receiving a clinical prediction result of a patient from the ensemble prediction systems 120 established in the external medical institutions, and a CDSS interface unit for communicating with the clinical decision supporting system 110.

When the decision-making ensemble prediction unit 121 receives, from the external medical institutions, a request for clinical prediction of a specific patient, the decision-making ensemble prediction unit 121 outputs a clinical prediction result of the patient using a clinical prediction model generated by the machine learning engine 140.

The decision-making ensemble prediction unit 121 transmits the output clinical prediction result to the medical institutions which have made the request, wherein the clinical prediction result is output on the basis of medical information learning big data obtained by converting, into a common format, hospital clinical information and lifelog information generated, accumulated, and managed autonomously.

Furthermore, the decision-making ensemble prediction unit 121 may send a request for clinical prediction of a specific patient to the external medical institutions, and at this time, the decision-making ensemble prediction unit 121 may provide clinical information from which personal information of the patient has been deleted.

When the results of the requested clinical prediction are received from the external medical institutions, the decision-making ensemble prediction unit 121 integrates the received results with a clinical prediction result output autonomously by the decision-making ensemble prediction unit 121.

The ensemble prediction represents a prediction of a future progress of an illness of the specific patient, and enables output of a correct and reliable ensemble prediction result by integrating the clinical prediction results received from the external medical institutions with the clinical prediction result output autonomously. In this manner, new information about the illness discovered by the external medical institutions may be obtained, and an ensemble prediction result may be output with improved accuracy and reliability by integrating a plurality of prediction results. That is, the decision-making ensemble prediction unit 121 performs an ensemble prediction by integrating a clinical prediction result obtained through machine learning based on own medical information learning big data of a specific medical institution with clinical prediction results obtained through machine learning based on medical information learning big data of one or more external medical institutions, and provides an ensemble prediction result to the clinical decision supporting system 110, so that a decision of a medical person may be made with improved accuracy and reliability.

The external medical institutions may transmit, to the medical institution which has made the clinical prediction request, opinions of specialized medical persons of the external medical institutions together with clinical prediction results generated autonomously by the external medical institutions, so that a medical person of the medical institution which has made the clinical prediction request may provide a correct medical service to a patient by integrating the opinions of the specialized medical persons.

The decision-making ensemble prediction unit 121 transmits the output ensemble prediction result to the clinical decision supporting system 110, so that the clinical decision supporting system 110 may generate clinical decision information so that the medical person may correctly make a decision.

The cooperative hospital management unit 122 stores, updates, or deletes hospital information for cooperation between the medical institution and the external medical institutions, or manages a combination of the hospital information, wherein the hospital information includes various pieces of information including a specialized field of each medical institution, a network address of each medical institution, a location of each medical institution, a size of each medical institution, or a combination thereof.

The ensemble prediction system 120 requests or receives a clinical prediction result for a specific patient through the other-institution interface.

The other-institution interface may include a plurality of virtual interface to directly communicate with each external medical institution, or may include a single communication interface to communicate with the external medical institutions.

The CDSS interface is used to transmit or receive data between the ensemble prediction system 120 and the clinical decision supporting system 110.

Furthermore, the clinical decision supporting system 110 generates and provides clinical decision information which is an overall guideline on a treatment of a patient, a treatment strategy, a prescription, prevention, and a method of managing a future progress of an illness of the patient, on the basis of an ensemble prediction result received from the ensemble prediction system 120.

The clinical decision information may be generated in a knowledge-based manner, or may be generated in a non-knowledge-based manner.

The clinical decision information may be based on knowledge, or may be based on a decision tree, a neural network, naive Bayes, or a combination thereof, or may be provided by a combination thereof.

According to the knowledge base, the decision information is generated on the basis of a specific rule set by an operator of the clinical decision supporting ensemble system 100, wherein the rule may be differently set by the operator.

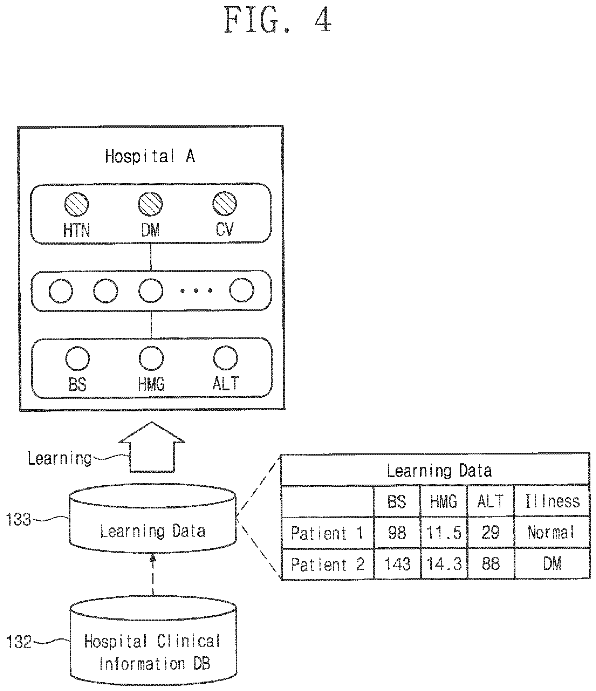

FIG. 4 is a diagram illustrating a process of performing machine learning through a machine learning engine in each medical institution according to an embodiment of the inventive concept.

As illustrated in FIG. 4, machine learning is performed through the machine learning engine 140 provided to each medical institution.

In the process of performing machine learning, clinical information data for each person is extracted from the clinical information DB 132 of a corresponding medical institution to generate learning data for machine learning.

The generated learning data is stored in the medical information learning big data DB 133 of each hospital, and is used as input data for machine learning.

The learning data may include numerical values (e.g., blood glucose, hemoglobin (HMG)) for each health feature of each person, liver function indices (e.g., alanine transaminase (ALT)), and illness information (e.g., existence or non-existence of an illness).

Furthermore, it would be obvious that personal information (e.g., e.g.. an address, a resident registration number, a phone number, etc.) is not included in the learning data since the learning data is generated on the basis of clinical data of patients accumulated and stored in each medical institution.

The learning data may be generated on the basis of not only the clinical data accumulated in each medical institution but also public cohort clinical data provided from National Health Insurance Service or Health Insurance Review & Assessment Service, or may be provided from another medical institution or a medical information providing institution.

Next, a deep learning network structure for an illness prediction of a corresponding hospital is designed. The structure of the deep learning network may be differently designed according to characteristics of each hospital, such as a specialized field (e.g., cancer, internal medicine, pediatrics, etc.) or majority types of patients.

Next, the learning data is input to the designed deep learning network so that the deep learning network may learn the learning data.

A final output of the deep learning network is a clinical prediction result obtained by predicting a future progress of a specific illness (e.g., high blood pressure, diabetes, cardiovascular disorders, etc.).

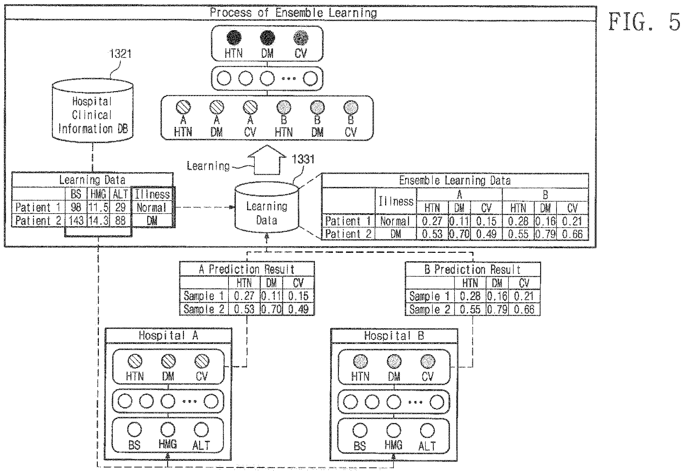

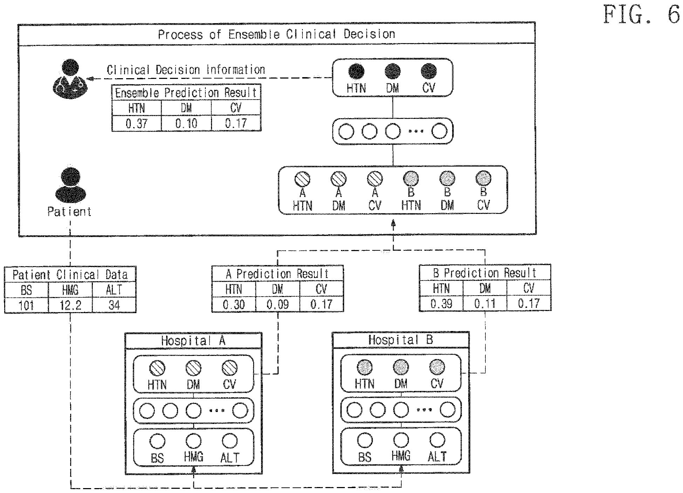

Described below with reference to FIGS. 5 and 6 is a process of performing ensemble learning for an ensemble prediction, and a process of outputting an ensemble clinical prediction result for a specific patient and proving clinical decision information including the ensemble clinical prediction result according to another embodiment of the inventive concept.

FIG. 5 is a diagram illustrating a process of performing ensemble learning for an ensemble prediction in a clinical decision supporting ensemble system according to another embodiment of the inventive concept.

A clinical decision supporting ensemble system 1001 may generate an ensemble clinical prediction result through machine learning in the same manner as performed in each hospital.

As illustrated in FIG. 5, the clinical decision supporting ensemble system 1001, which performs ensemble learning to generate an ensemble clinical prediction result for a specific patient, extracts clinical information data for a plurality of patients from a hospital clinical information DB 1321 to generate learning data for each patient.

The generated learning data for each patient is stored in an ensemble learning data DB 1331.

As illustrated in FIG. 5, the learning data may be configured as a matrix or a number-type vector, and includes numerical values for each health feature and illness information (which maybe represented by a class or a label).

Next, the clinical decision supporting ensemble system 1001 transmits the generated learning data for each patient to a plurality of other medical institutions or medical information providing institutions to request a prediction result for the learning data.

Here, the learning data (vector or matrix form) to be transmitted includes numerical values for each health feature excepting illness information, and personal information of patients is deleted from the learning data so as to be replaced with other identifiers (e.g., ID).

Next, each medical institution or medical information providing institution, which has received the learning data from the clinical decision supporting ensemble system 1001, outputs prediction result data by inputting the received learning data to a deep learning network (e.g., a prediction model) trained with learning data of each hospital through a deep-learning machine learning process in each medical institution or medical information providing institution.

Thereafter, each medical institution or medical information providing institution transmits the output prediction result data to the clinical decision supporting ensemble system 1001 which has requested the prediction result.

The prediction result data transmitted from each medical institution or medical information providing institution is an output value of a prediction model of each medical institution or medical information providing institution, and may be configured in the form of a number-type vector. The number-type vector may be presented as the degree of danger (probability vector) for each disease, and may be an abstracted number vector which is not 1:1 mapped to a specific disease due to characteristics of deep learning (machine learning).

Next, the clinical decision supporting ensemble system 1001, which has received the prediction results from each medical institution or medical information providing institution, integrates the prediction results received from each medical institution or medical information providing institution with a prediction result generated autonomously by the clinical decision supporting ensemble system 1001, and extracts final ensemble learning data on the basis of the integrated prediction results to store the final ensemble learning data in the ensemble learning data DB 1331.

The generated ensemble learning data includes numerical values for each health feature of each patient, illness information, and a prediction result of each hospital.

Next, the clinical decision supporting ensemble system 1001 designs an ensemble deep learning network structure for ensemble learning.

Designing of the ensemble deep learning network structure includes setting of a dimension of input data, a dimension of output data, the number of layers of the deep learning network, the number of nodes in each deep learning network layer, and deep learning parameters (representing parameters of a typical neural network or deep learning, such as a learning rate, a min-batch size, the type of an activation function, etc.).

Next, an ensemble prediction system 1201 performs pre-training for the ensemble deep learning network using the ensemble learning data.

Data input to the ensemble deep learning network for the purpose of the pre-training is a prediction result of each medical institution or medical information providing institution according to each patient, included in the ensemble learning data.

The pre-training represents learning in advance weights in the ensemble deep learning network so as to match up with characteristics of data by using the data which lacks illness information, and is performed to prevent overfitting of the ensemble deep learning network due to learning data.

The pre-training may be performed using a contrastive divergence (CD) technique for each layer of the ensemble deep learning network on the basis of a restricted Boltzmann machine (RMB) which is one of machine learning methods. However, an embodiment of the inventive concept is not limited to the RMB or the CD.

Next, the clinical decision supporting ensemble system 1001 performs fine tuning on the ensemble deep learning network using the ensemble learning data.

Data input for the fine tuning is a result of analysis by each medical institution or medical information providing institution according to each patient and illness information according to each patient.

The fine tuning is performed to readjust the weights of the ensemble deep learning network for which the pre-training has been performed, so as to match up with characteristics of data input to perform a prediction on an illness.

Although the ensemble learning processes are described with reference to FIG. 5 individually for a hospital A and a hospital B, the ensemble learning processes may be performed in the hospital A or the hospital B, or may be performed in another hospital (e.g., a hospital C).

FIG. 5 illustrates that ensemble learning is performed on the basis of prediction results for two hospitals (the hospitals A and B), but the number of hospitals (i.e., medical institutions or medical information providing institutions) is not limited.

FIG. 6 is a diagram illustrating a process of outputting an ensemble prediction result for a specific patient through ensemble learning to provide clinical decision information to a user according to another embodiment of the inventive concept.

As illustrated in FIG. 6, in the process of outputting an ensemble prediction result for a specific patient through ensemble learning to provide clinical decision information to a user, clinical data of the specific patient is transmitted to another medical institution or medical information providing institution to request a prediction result.

Illness information and personal information of the patient haven been deleted from the clinical data of the patient transmitted to the other medical institution or medical information providing institution.

Each medical institution or medical information providing institution, which has received the clinical data of the specific patient, inputs the clinical data of the specific patient to a clinical prediction model (e.g., a deep learning network) established autonomously in each medical institution or medical information providing institution to output a prediction result for the patient, and transmits the prediction result to the clinical decision supporting ensemble system 1001 which has requested the prediction result.

The prediction result of each medical institution or medical information providing institution may be a value of the degree of danger (probability) for each disease type.

Next, the clinical decision supporting ensemble system 1001 integrates the prediction results received from a plurality of medical institutions with its own prediction result obtained using the clinical data of the specific patient, and inputs the integrated prediction results to the ensemble deep learning network to output an ensemble prediction result.

Furthermore, the clinical decision supporting ensemble system 1001 connects the prediction results received from a plurality of medical institutions or medical information providing institutions to its own prediction result to form a single vector, and inputs the vector to the ensemble deep learning network to output an ensemble prediction result.

A final result of the ensemble deep learning network may include the degree of danger (probability vector) for each disease. This may vary with a configuration of the deep learning network. For example, in the case where the deep learning network and the ensemble deep learning networks are established for each disease, final clinical decision information only provides the degree of danger (probability value) for a corresponding disease.

Next, the clinical decision supporting ensemble system 1001 transmits clinical decision information including a final result to a terminal of a user (medical person) so that the user may use the clinical decision information.

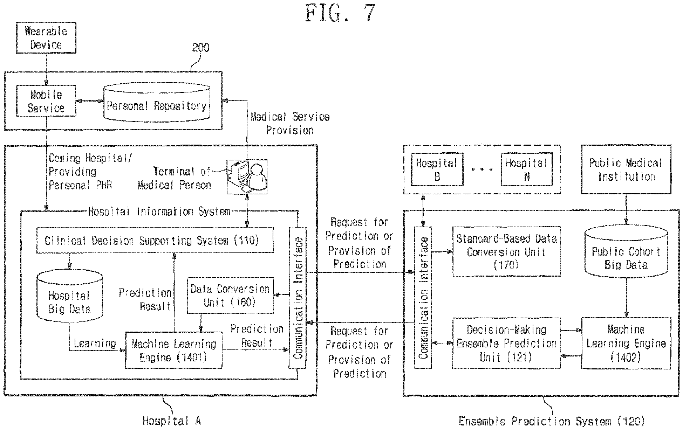

FIG. 7 is a block diagram illustrating a configuration of a clinical decision supporting ensemble system according to another embodiment of the inventive concept.

As illustrated in FIG. 7, unlike the clinical decision supporting ensemble system 100 described above with reference to FIGS. 1 to 3, the clinical decision supporting system 110 of the clinical decision supporting ensemble system 100 may be configured as an element of a hospital information system of a medical institution (e.g., a hospital), and the ensemble prediction system 120 may be configured as a platform or a server on the Internet.

The hospital information system represents a system which includes the clinical information system 150 to manage overall information of a hospital such as financial or accounting information.

A machine learning engine 1401 of the medical institution performs learning by using hospital big data managed in the hospital information system, wherein the hospital big data includes a PHR of a patient, lifelog information, an EMR, a medical image, or a combination thereof. That is, the machine learning engine 1401 integrally learns PHRs, lifelog information, EMRs, medical images, and the like of a plurality of patients to generate at least one clinical prediction model, and outputs a clinical prediction result for a specific patient through the clinical prediction model.

When a medical person requests clinical decision information of a specific patient from the clinical decision supporting system 110, the hospital information system provides clinical information, from which personal information of the specific patient has been deleted, to the ensemble prediction system 120 on the Internet via a communication interface to request a clinical prediction result.

Thereafter, when the requested clinical prediction result is received from the ensemble prediction system 120, the clinical prediction result is converted into a common data format used in the corresponding medical institution through a data conversion unit 160 so as to be provided to the machine learning engine 1401, and the machine learning engine 1401 provides, to the clinical decision supporting system 110, the clinical prediction result of the ensemble prediction system 120 and a clinical prediction result generated autonomously.

Furthermore, the clinical decision supporting system 110 provides the clinical decision information to the medical person on the basis of the received clinical prediction results.

The ensemble prediction system 120 on the Internet may include a standard-based data conversion unit 170, the decision-making ensemble prediction unit 121, and a machine learning engine 1402.

When the ensemble prediction system 120 receives the request for the clinical prediction result and the clinical information of the patient from the hospital information system, the ensemble prediction system 120 converts the clinical information into a standard format through the standard-based data conversion unit 170. The standard format is VMR, eVMR, or Arden syntax of the HL7 standard.

The decision-making ensemble prediction unit 121 provides the converted clinical information of the patient to the machine learning engine 1402, and the machine learning engine 1402 inputs the clinical information of the patient to a pre-extracted clinical prediction model to output a clinical prediction result for the patient, and provides the clinical prediction result to the decision-making ensemble prediction unit 121.

The machine learning engine 1402 learns on the basis of public cohort big data to extract a clinical prediction model, wherein the public cohort big data represents public clinical information provided periodically from a public medical institution. In this manner, the machine learning engine 1402 may learn a large amount of public clinical information, and may output a correct and reliable clinical prediction result. Since the learning has been described with reference to FIG. 3, detailed descriptions of the learning are not provided.

Furthermore, the decision-making ensemble prediction unit 121 performs an ensemble prediction on the basis of the clinical information of the patient received from the medical institution and the clinical prediction result, and provides an ensemble prediction result to the medical institution which has requested the prediction result.

When the ensemble prediction system 120 receives a request for a clinical prediction result from a specific medical institution, the ensemble prediction system 120 may request the clinical prediction result for the patient from a plurality of external medical institutions excepting the specific medical institution, and may integrate clinical prediction results received from the external medical institutions with a clinical prediction result output based on the public cohort big data to perform a decision-making ensemble prediction.

As described above with reference to FIG. 7, the clinical decision supporting ensemble system 100 according to an embodiment of the inventive concept may be implemented in various forms, and may be applicable in various fields in which decision making is required.

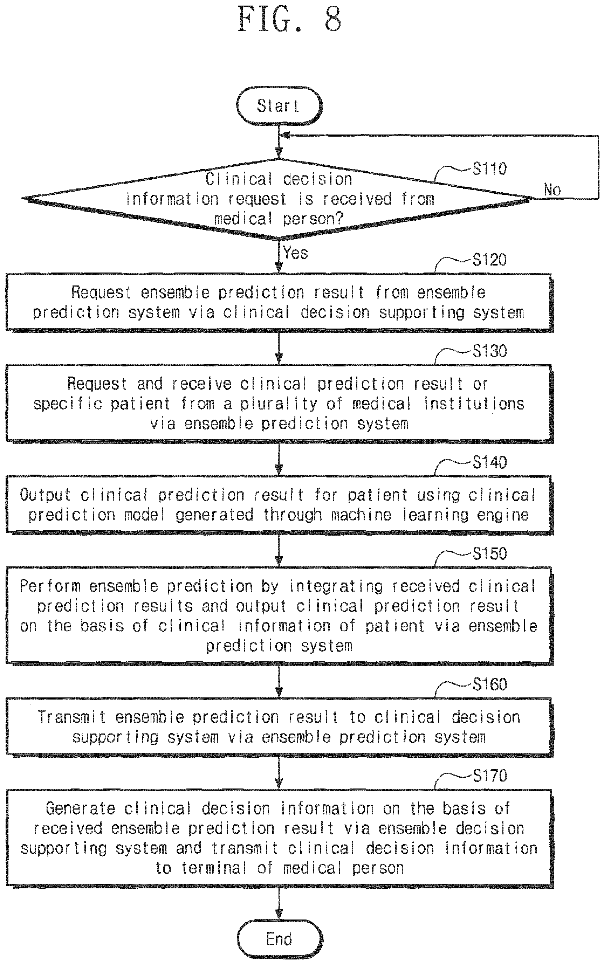

FIG. 8 is a flowchart illustrating a procedure of generating clinical decision information to provide the clinical decision information to a terminal of a medical person according to an embodiment of the inventive concept.

As illustrated in FIG. 8, in the procedure of generating clinical decision information for a specific patient to provide the clinical decision information to a medical person, when the medical person requests the clinical decision information for the specific patient (S110), a request for an ensemble prediction result is sent to the ensemble prediction system 120 via the clinical decision supporting system 110 (S120).

The clinical decision supporting system 110 provides, to the medical person, the clinical decision information which is a guideline on medical activities such as diagnosis on the specific patient, a prescription, or a treatment method, and may be established in various manners such as a knowledge-based manner or a non-knowledge-based manner according to an operator of the clinical decision supporting ensemble system 100.

Next, a clinical prediction result for the patient is requested and received from a plurality of external medical institutions via the ensemble prediction system 120 (S130).

When requesting the clinical prediction result from the external medical institutions, clinical information from which personal information of the patient has been deleted is provided to the external medical institutions, and each external medical institution autonomously generates the clinical prediction result for the patient and transmits the clinical prediction result to the medical institution which has requested the clinical prediction result.

The clinical prediction result is output through a clinical prediction model extracted by learning medical information learning big data in the machine learning engine 140 of the clinical decision ensemble supporting system 100 established in each medical institution.

Next, a clinical prediction result for the patient is output using the clinical prediction model generated through the machine learning engine 140 (S140).

The clinical prediction results of operation S130 represent the clinical prediction results generated in the external medical institutions, and the clinical prediction result of operation S140 represents the clinical prediction result generated in the medical institution which has requested the clinical prediction results from the external medical institutions.

Next, the received clinical prediction results and the clinical prediction result generated autonomously are integrated on the basis of the clinical information of the patient to perform an ensemble prediction using the ensemble prediction system 120 (S150).

The ensemble prediction is performed so that the clinical prediction results generated in a plurality of medical institutions and the clinical prediction result generated in a corresponding medical institution are integrated through interoperation therebetween, and on the basis of the integrated clinical prediction results, a current condition of a patient and a future progress of an illness of the patient may be correctly predicted so that the medical person may correctly and quickly perform medical activities on the patient.

Next, the ensemble prediction result is transmitted to the clinical decision supporting system 110 via the ensemble prediction system 120 (S160), and the clinical decision supporting system 110 generates clinical decision information for the patient on the basis of the received ensemble prediction result to transmit the clinical decision information to a terminal of the medical person (S170).

The clinical decision information is a guideline including information about a current condition of the patient, a diagnosis, prediction, prescription, treatment strategy, or management method regarding a future progress of an illness, or a combination thereof.

As described above, the clinical decision supporting ensemble system according to an embodiment of the inventive concept interworks with a plurality of medical institutions to perform a clinical prediction for a specific patient to predict a current condition of the patient and a future progress of an illness with improved accuracy and reliability, and supports a clinical decision about a diagnosis, prescription, treatment strategy, or management method regarding the current condition of the patient and the future progress of the illness so as to assist a medical person in performing medical activities correctly and quickly.

According to embodiments of the inventive concept, interoperation with a plurality of medical devices is performed through a common ensemble prediction system, clinical prediction results for a specific patient respectively provided from the medical institutions are integrated, and a diagnosis on the patient, to which characteristics of the patient and the integrated clinical prediction results are reflected, is performed and a future progress of a condition of the patient is predicted, so as to provide, to a medical person, a guideline on a prediction, diagnosis, prevention, treatment strategy, prior warning on an emergency situation, prescription, or efficient illness management regarding the condition of the patient, so that the medical person may make a decision about medical activities quickly and correctly.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed.
Further implementations are summarized in the following examples:
Example 1. A clinical decision supporting ensemble system configured to provide clinical decision information by performing an ensemble prediction by integrating clinical prediction results provided from a plurality of medical institutions, medical information providing institutions, or combinations thereof.
Example 2. The clinical decision supporting ensemble system of example 1, wherein the ensemble prediction is performed on the basis of the clinical prediction results obtained through machine learning using medical information learning big data of each of the plurality of medical institutions.
Example 3. The clinical decision supporting ensemble system of example 2, wherein the ensemble prediction supports a clinical decision by integrating a clinical prediction result based on machine learning of own medical information learning big data of a specific medical institution and clinical prediction results based on machine learning of medical information learning big data of each of one or more external medical institutions.
Example 4. The clinical decision supporting ensemble system of example 1, 2 or 3 wherein the clinical decision information is provided by:
   a knowledge base;
   a decision tree, a neural network, naive Bayes, or a combination thereof according to each person, illness, or a combination thereof; or
   a combination thereof.
Example 5. The clinical decision supporting ensemble system of one of examples 2 to 4, wherein the machine learning comprises an artificial intelligence technique for performing learning, inference, prediction, or a combination thereof in order to multi-dimensionally analyze big data comprising a medical record, a lifelog, or a combination thereof for a patient-centered medical service.
Example 6. The clinical decision supporting ensemble system of one of examples 2 to 5, wherein the machine learning is performed to output, as a clinical prediction result, highly reliable numerical information extracted or integrated through speeding up by a parallel cluster and quantization of medical information learning big data.
Example 7. A clinical decision supporting ensemble system comprising:
   a clinical decision supporting system configured to provide clinical decision information in response to a clinical decision request of a user; and
   an ensemble prediction system configured to provide a clinical prediction result in response to a request of the clinical decision supporting system,
   wherein the ensemble prediction system performs an ensemble prediction for a decision by integrating clinical prediction results provided from a plurality of medical institutions, medical information providing institutions, or combinations thereof.
Example 8. The clinical decision supporting ensemble system of example 7, further comprising a machine learning engine comprising a deep learning algorithm for performing learning by using big data from a clinical information database.
Example 9. The clinical decision supporting ensemble system of example 8, wherein the clinical information database comprises hospital clinical information of an individual hospital and lifelog information of an outpatient.
Example 10. The clinical decision supporting ensemble system of example 8 or 9, wherein the machine learning engine abstracts features of medical information from big data comprising an electronic medical record (EMR), a personal health record (PHR), a medical image, lifelog information, or a combination thereof, and extracts a prediction model by learning the big data to early predict a dangerous situation of a disease, thereby improving reliability of a clinical prediction result.
Example 11. The clinical decision supporting ensemble system of one of examples 7 to 10, wherein the ensemble prediction system comprises:
   an interface configured to send a prediction request to ensemble prediction systems present in a plurality of external medical institutions and receive prediction results from the external medical institutions; and
   a cooperative hospital management unit configured to manage hospital information for cooperating with the external medical institutions.
Example 12. The clinical decision supporting ensemble system of one of examples 7 to 11, wherein the institutions are provided with the ensemble prediction system, a machine learning engine, and medical information learning big data.
Example 13. The clinical decision supporting ensemble system of one of examples 7 to 12, wherein the clinical decision supporting ensemble system collects biometric data of a patient comprising a lifelog of the patient to continuously monitor and manage a condition of the patient, and provides the condition of the patient to an loT device.
Examples 14. A clinical decision supporting method comprising:
   performing an ensemble prediction by integrating a plurality of clinical prediction results; and
   providing clinical decision information with improved accuracy through the ensemble prediction.
Example 15. The method of example 14, wherein the ensemble prediction is performed on the basis of the clinical prediction results obtained through machine learning using medical information learning big data of each of a plurality of medical institutions.
Example 16. The method of example 15, wherein the ensemble prediction supports a clinical decision by integrating a clinical prediction result based on machine learning of own medical information learning big data of a specific medical institution and clinical prediction results based on machine learning of medical information learning big data of each of one or more external medical institutions.
Example 17. The method of one of examples 14 to 16, wherein the clinical decision information is provided by:
   a knowledge base;
   a decision tree, a neural network, naive Bayes, or a combination thereof according to each person, illness, or a combination thereof; or
   a combination thereof.
Example 18. The method of one of examples 15 to 17, wherein the machine learning comprises an artificial intelligence technique for performing learning, inference, prediction, or a combination thereof in order to multi-dimensionally analyze big data comprising a medical record, a lifelog, or a combination thereof for a patient-centered medical service.
Example 19. The method of one of examples 15 to 18, wherein the machine learning is performed to output, as a clinical prediction result, highly reliable numerical information extracted or integrated through speeding up by a parallel cluster and quantization of medical information learning big data.
Examples 20. A clinical decision supporting method comprising:
   providing clinical decision information in response to a clinical decision request of a user via a clinical decision supporting system; and
   providing a clinical prediction result in response to a request of the clinical decision supporting system via an ensemble prediction system,
   wherein the ensemble prediction system performs an ensemble prediction for a decision by integrating clinical prediction results provided from a plurality of medical institutions, medical information providing institutions, or combinations thereof.
Example 21. The method of example 20, further comprising performing learning by using big data from a clinical information database through a machine learning engine.
Example 22. The method of example 21, wherein the clinical information database comprises hospital clinical information of an individual hospital and lifelog information of an outpatient.
Example 23. The method of example 21 or 22, wherein the machine learning engine abstracts features of medical information from big data comprising an electronic medical record (EMR), a personal health record (PHR), a medical image, lifelog information, or a combination thereof, and extracts a clinical prediction model by learning the big data to early predict a dangerous situation of a disease, thereby improving reliability of a clinical prediction result.
Example 24. The method of one of examples 20 to 23, wherein the ensemble prediction system comprises:
   an interface configured to send a prediction request to ensemble prediction systems present in a plurality of external medical institutions and receive prediction results from the external medical institutions; and
   a cooperative hospital management unit configured to manage hospital information for cooperating with the external medical institutions.
Example 25. The method of one of examples 20 to 24, wherein the institutions are provided with the ensemble prediction system, a machine learning engine, and medical information learning big data.
Examples 26. The method of one of examples 20 to 25, comprising collecting biometric data of a patient comprising lifelog information of the patient to continuously monitor and manage a condition of the patient, and providing the condition of the patient to an loT device.
Example 27. A clinical decision supporting method comprising receiving prediction results generated by other medical institutions or medical information providing institutions by using, as an input, learning data provided from a specific medical institution or medical information providing institution, integrating the received prediction results and a prediction result generated autonomously by the specific medical institution or medical information providing institution to extract ensemble learning data, and performing ensemble learning by using the extracted ensemble learning data.
Example 28. The method of example 27, wherein the receiving the prediction results comprises:
   generating, by the specific medical institution or medical information providing institution, learning data to provide the learning data to the other medical institutions or medical information providing institutions; and
   providing, to the specific medical institution or medical information providing institution, the prediction results obtained using the learning data as the input to a prediction model of the other medical institutions or medical information providing institutions.
Example 29. The method of example 27 or 28, wherein the ensemble learning comprises:
   performing pre-training by using the prediction results of each of the other medical institutions or medical information providing institutions; and
   performing fine tuning by using illness information in addition to the prediction results.
Example 30. A clinical decision supporting method comprising integrating an own prediction result generated by a specific medical institution or medical information providing institution by using clinical data of a specific patient and prediction results generated by other medical institutions or medical information providing institutions by using the clinical data of the specific patient as an input, and performing an ensemble prediction using the integrated prediction results.
Example 31. A clinical decision supporting ensemble system configured to receive prediction results generated by other medical institutions or medical information providing institutions by using, as an input, learning data provided from a specific medical institution or medical information providing institution, integrate the received prediction results and a prediction result generated autonomously by the specific medical institution or medical information providing institution to extract ensemble learning data, and perform ensemble learning by using the extracted ensemble learning data.
Example 32. The clinical decision supporting ensemble system of example 31, wherein the receiving the prediction results comprises:
   generating, by the specific medical institution or medical information providing institution, learning data to provide the learning data to the other medical institutions or medical information providing institutions; and
   providing, to the specific medical institution or medical information providing institution, the prediction results obtained using the learning data as the input to a prediction model of the other medical institutions or medical information providing institutions.
Example 33. The clinical decision supporting ensemble system of example 31 or 32, wherein the ensemble learning comprises:
   performing pre-training by using the prediction results of each of the other medical institutions or medical information providing institutions; and
   performing fine tuning by using illness information in addition to the prediction results.
Example 34. A clinical decision supporting ensemble system configured to integrate an own prediction result generated by a specific medical institution or medical information providing institution by using clinical data of a specific patient and prediction results generated by other medical institutions or medical information providing institutions by using the clinical data of the specific patient as an input, and perform an ensemble prediction using the integrated prediction results.

## Claims

1. A clinical decision supporting ensemble system configured to provide clinical decision information by performing an ensemble prediction by integrating clinical prediction results provided from a plurality of medical institutions, medical information providing institutions, or combinations thereof.

2. The clinical decision supporting ensemble system of claim 1, wherein the ensemble prediction is performed on the basis of the clinical prediction results obtained through machine learning using medical information learning big data of each of the plurality of medical institutions.

3. The clinical decision supporting ensemble system of claim 2, wherein the ensemble prediction supports a clinical decision by integrating a clinical prediction result based on machine learning of own medical information learning big data of a specific medical institution and clinical prediction results based on machine learning of medical information learning big data of each of one or more external medical institutions.

4. The clinical decision supporting ensemble system of claim 1, 2, or 3 wherein the clinical decision information is provided by:
a knowledge base;
a decision tree, a neural network, naive Bayes, or a combination thereof according to each person, illness, or a combination thereof; or
a combination thereof.

5. The clinical decision supporting ensemble system of one of claims 2 to 4, wherein the machine learning comprises an artificial intelligence technique for performing learning, inference, prediction, or a combination thereof in order to multi-dimensionally analyze big data comprising a medical record, a lifelog, or a combination thereof for a patient-centered medical service.

6. The clinical decision supporting ensemble system of one of claims 2 to 5, wherein the machine learning is performed to output, as a clinical prediction result, highly reliable numerical information extracted or integrated through speeding up by a parallel cluster and quantization of medical information learning big data.

7. A clinical decision supporting ensemble system comprising:
a clinical decision supporting system configured to provide clinical decision information in response to a clinical decision request of a user; and
an ensemble prediction system configured to provide a clinical prediction result in response to a request of the clinical decision supporting system,
wherein the ensemble prediction system performs an ensemble prediction for a decision by integrating clinical prediction results provided from a plurality of medical institutions, medical information providing institutions, or combinations thereof.

8. The clinical decision supporting ensemble system of claim 7, further comprising a machine learning engine comprising a deep learning algorithm for performing learning by using big data from a clinical information database.

9. The clinical decision supporting ensemble system of claim 8, wherein the clinical information database comprises hospital clinical information of an individual hospital and lifelog information of an outpatient.

10. The clinical decision supporting ensemble system of claim 8 or 9, wherein the machine learning engine abstracts features of medical information from big data comprising an electronic medical record (EMR), a personal health record (PHR), a medical image, lifelog information, or a combination thereof, and extracts a prediction model by learning the big data to early predict a dangerous situation of a disease, thereby improving reliability of a clinical prediction result.

11. The clinical decision supporting ensemble system of one of claims 7 to 10, wherein the ensemble prediction system comprises:
an interface configured to send a prediction request to ensemble prediction systems present in a plurality of external medical institutions and receive prediction results from the external medical institutions; and
a cooperative hospital management unit configured to manage hospital information for cooperating with the external medical institutions.

12. The clinical decision supporting ensemble system one of claims 7 to 11, wherein the institutions are provided with the ensemble prediction system, a machine learning engine, and medical information learning big data.

13. The clinical decision supporting ensemble system of one of claims 7 to 12, wherein the clinical decision supporting ensemble system collects biometric data of a patient comprising a lifelog of the patient to continuously monitor and manage a condition of the patient, and provides the condition of the patient to an IoT device.

14. A clinical decision supporting method comprising:
performing an ensemble prediction by integrating a plurality of clinical prediction results; and
providing clinical decision information with improved accuracy through the ensemble prediction.

15. A clinical decision supporting method comprising:
providing clinical decision information in response to a clinical decision request of a user via a clinical decision supporting system; and
providing a clinical prediction result in response to a request of the clinical decision supporting system via an ensemble prediction system,
wherein the ensemble prediction system performs an ensemble prediction for a decision by integrating clinical prediction results provided from a plurality of medical institutions, medical information providing institutions, or combinations thereof.
